# EUROPEAN PATENT SPECIFICATION

(11) **EP 4 232 268 B1**
(45) Date of publication and mention of the grant of the patent: **23.09.2026**
(21) Application number: 21810210.1
(22) Date of filing: 22.10.2021
(51) Int. Cl.: B29C 65/16, B29C 65/66, B29C 65/68, A61M 25/00, A61M 25/10, B23K 13/01, B23K 26/244, B23K 26/282, B23K 26/324

(54) **WELDING METHOD USING FIBER LASER FOR COMPONENTS OF A MEDICAL DEVICE AND SYSTEM FOR FORMING A MEDICAL DEVICE**
SCHWEISSVERFAHREN MIT FASERLASER FÜR KOMPONENTEN EINER MEDIZINISCHEN VORRICHTUNG UND SYSTEM ZUR HERSTELLUNG EINER MEDIZINISCHEN VORRICHTUNG
PROCÉDÉ DE SOUDAGE À L'AIDE D'UN LASER À FIBRE POUR COMPOSANTS D'UN DISPOSITIF MÉDICAL ET SYSTÈME POUR FORMER UN DISPOSITIF MÉDICAL

(30) Priority: 23.10.2020 US 202063104782 P
(43) Date of publication of application: 30.08.2023
(73) Proprietor: Medtronic, Inc., Minneapolis, Minnesota 55432 (US)
(72) Inventor: PISCHLAR, Jesse J., Mounds View, Minnesota 55112 (US); HEILE, Elizabeth A., Mounds View, Minnesota 55112 (US)
(74) Representative: Zimmermann & Partner Patentanwälte mbB
(86) International application number: PCT/US2021/056326
(87) International publication number: WO 2022/087468

(56) References cited:
- EP-A2- 1 234 595
- WO-A1-93/10961

## Description

### TECHNICAL FIELD

The disclosure relates to methods of welding for components of medical devices, particularly thermally welding polymeric medical device components.

### BACKGROUND

Laser welding may be used to produce welds for items such as medical devices and related components. In some examples, laser welding may provide hermetic seals for medical devices enclosures and associated components for the medical devices. EP 1 234 595 A2 describes through-transmission welding of catheter components. WO 93/10961 describes laser bonding of angioplasty balloon catheters.

### SUMMARY

The disclosure is directed to systems and techniques for thermally welding of a first polymeric member with a second polymeric member at a joint region using a compression sleeve to provide a smooth, e.g., tapered, joint and a strong, hermetic bond between the first and second polymeric members. The thermally welding is controlled to selectively heat the joint region between the first polymeric member and the second polymeric member.

In one aspect, the invention is directed to a system for forming a medical device according to claim 1.

In another aspect, the invention is directed to a method for forming a medical device according to claim 10.

The details of one or more examples of the disclosure are set forth in the accompanying drawings and the description below. Other features, objects, and advantages of the disclosure will be apparent from the description and drawings, and from the claims.

### BRIEF DESCRIPTION OF DRAWINGS

FIG. 1A is a conceptual diagram that illustrates an example of a welding system that may be used to form an example medical device in accordance with aspects of this disclosure.
FIG. 1B is a conceptual diagram that illustrates an example of the welding system of FIG. 1A operating in a direct welding mode.
FIG. 1C is a conceptual diagram that illustrates an example of the welding system of FIG. 1A operating in a transmission welding mode.
FIG. 2 is a conceptual diagram that illustrates a view of an example medical device, including a first member and a second member, in accordance with aspects of this disclosure.
FIGS. 3A-3D are conceptual diagrams that illustrate example cross-sections of a joint region during an example thermal welding process in accordance with aspects of this disclosure.
FIG. 4 is a flowchart illustrating an example method of welding a medical balloon of a medical device to a shaft of the medical device in accordance with aspects of this disclosure.
FIG. 5 is a flowchart illustrating an example method of welding a first polymeric member to a second polymeric member in accordance with aspects of this disclosure.
FIG. 6A is a plan view of a medical device that includes a handle with an irrigation tube, an extension tube, and a luer.
FIG. 6B is a schematic illustration of a process for bonding an extension tube on the irrigation tube of the medical device of FIG. 6A.
FIG. 6C is a schematic illustration of a process for bonding the extension tube of FIG. 6B to the luer of the medical device of FIG. 6A.
FIG. 7 is an example plot of absorption vs. wavelength for various components of a medical device.

### DETAILED DESCRIPTION

The disclosure is directed to systems and technique for welding of a first polymeric member to a second polymeric member at a joint region using a compression sleeve to providing a smooth joint region and a strong, hermetic bond between the two tubular members, e.g., to couple the first member and the second member via fiber laser welding. The disclosure also describes methods for forming medical devices. The described systems and technique may, during a welding process, reduce expansion and distortion of the neck of a medical balloon with high equator to neck ratio welded to a catheter body.

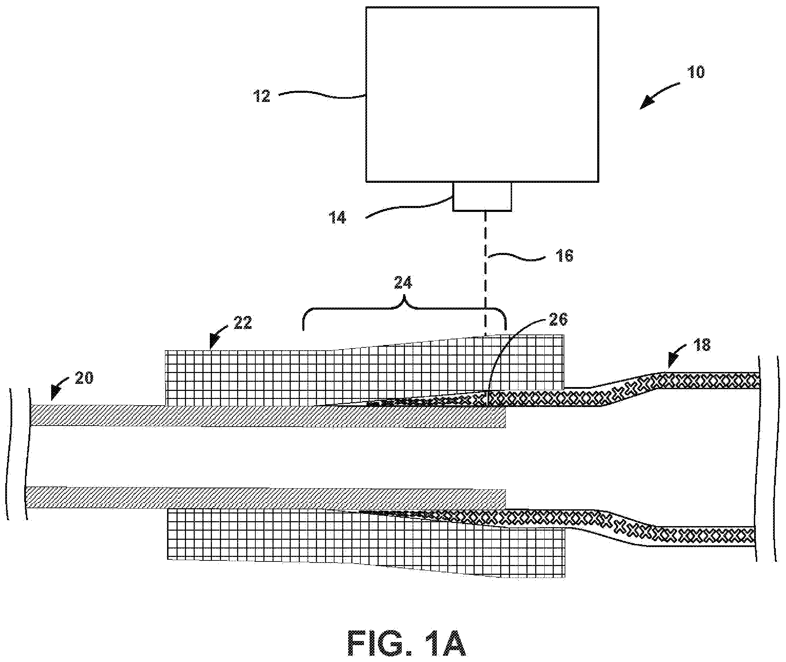

FIG. 1A is a conceptual diagram that illustrates an example of a welding system 10 that may be used to join polymeric components. In the example illustrated in FIG. 1A, welding system 10 includes an energy source 12, a lens 14, a first tubular member 18, a second tubular member 20, and a compression sleeve 22. Energy source 12 is configured to emit an energy beam 16.

System 10 may be used to weld first tubular member 18 to second tubular member 20 at a joint region 24. In particular, system 10 may be used to form a smooth joint region 24 to couple first tubular member 18 and second tubular member 20.

In some examples, first tubular member 18 is a component of a medical device 50. For example, first tubular member 18 may be a medical balloon of medical device 50, such as a medical balloon with a high equator-to-neck ratio. In another example, the first tubular member 18 may be medical tubing, or a portion thereof, used to transport a fluid in a medical procedure or a medical device. Suitable fluid-transporting medical tubing can include, for example, a catheter or an extension tube or connector that overlies at least a portion of the catheter. In another example, the first tubular member may be a medical device component with an opening such as an orifice, a slot, or a luer configured to accept medical tubing.

In some examples, first tubular member 18 may include a first polymer, such as an elastic polymer that expands under pressure. Examples of the first polymer include, but not limited to, polyethylene, polyethylene terephthalate (PET), polyamide , polyether block amide elastomer, polyether ester elastomer, polytetrafluorethylene (PTFE), polyurethane, polyester, silicone, polyvinyl chloride, polypropylene, polyurethanes, polyamides, latex, natural rubber, synthetic rubber, or the like. In some examples, the first polymer may be selected to substantially transmit radiation having a selected range of wavelengths. For example, the first polymer may be selected to substantially transmit radiation having a wavelength within a range from about 800 nanometers (nm) to about 3000 nm, such as within a range from about 1500 nm to about 2200 nm. Substantially transmit may include transmission of about 30% to about 70% of incident radiation, such as 60% of incident radiation or 70% of incident radiation.

In some examples, second tubular member 20 is a component of a medical device. For example, second tubular member 20 may be medical tubing such as a catheter body, an extension tube or connector overlying at least a portion of the catheter body, a rigid hub, or a luer of the medical device. In some examples, second tubular member 20 may include a second polymer, which may be the same material as, or a different material from, first tubular member 18.

Examples of the second polymer include, but are not limited to, polyethylene, polyethylene terephthalate (PET), polyamide, polyether block amide elastomer, polyether ester elastomer, polytetrafluorethylene (PTFE), polyurethane, polyester, silicone, polyvinyl chloride, polypropylene, polyurethanes, polyamides, latex, natural rubber, synthetic rubber, or the like. In some examples, the second polymer may be selected to substantially absorb radiation having a selected range of wavelengths. For example, the second polymer may be selected to substantially absorb radiation having a wavelength within a range from about 800 nanometers (nm) to about 3000 nm, such as within a range from about 1500 nm to about 2200 nm. Substantially absorb may include absorption of about 30% to about 70% of incident radiation, such as 60% of incident radiation or 70% of incident radiation. Absorptivity of second tubular member 20 may be adjusted by the incorporation of different additives, such as carbon black, indium tin oxide, or other materials selected to absorb a selected wavelength of radiation.

Compression sleeve 22 is configured to, during a welding process, compress at least a portion of first tubular member 18 against at least a portion of second tubular member 20. By compressing at least a portion of first tubular member 18, compression sleeve 22 may aid in connecting of first tubular member 18 with second tubular member 20. In some examples, compression sleeve 22 may be configured to compress the parts to be welded to constrain first tubular member 18 and second tubular member 20 in a substantially fixed position. In some examples, compression sleeve 22 may include a heat-shrinkable sleeve. In other examples, compression sleeve 22 may include a non-shrinkable tube with a tight fit to joint region 24.

In some examples, compression sleeve 22 may be heated to the desired preshrunk dimension using a fixture. The diameter of the fixture may be selected based on the diameter of second tubular member 20 such that compression sleeve 22 may be shrink down to about the diameter of second tubular member 20. After shrinking compression sleeve 22, compression sleeve 22 may be fitted to second tubular member 20 to connect second tubular member 20 to first tubular member 18. Pre-shrinking compression sleeve 22 protects first tubular member 18 and/or second tubular member 20 from the heat, and thereby potential distortion of first tubular member 18 and/or second tubular member 20, during heat shrinking of compression sleeve 22.

In some examples, compression sleeve 22 may be directly apply to second tubular member 20 and may be configured to, when heated to a selected temperature, shrink around the parts to be welded to constrain first tubular member 18 and second tubular member 20 in a substantially fixed position as a bonding aid.

In some examples, compression sleeve 22 is removable after the welding process. For example, compression sleeve 22 may be removed by any suitable means, such as, for example, cutting, peeling, laser etching, or other material removal techniques. In some examples, compression sleeve 40 may include other compression sleeves or compression devices, such as a cold-shrink sleeve, a compression wrap, or a clamp.

Compression sleeve 22 may include any suitable polymer. For example, compression sleeve 22 may include fluorinated ethylene propylene (FEP), ethylene tetrafluoroethylene (ETFE), polyvinylidene fluoride (PVDF), polytetrafluoroethylene (PTFE), perfluoroalkoxy (PFA), polyethylene terephthalate (PET), polyolefin, polyether block amide, silicone or other suitable material.

While not wishing to be bound by any theory, presently available evidence indicates that FEP materials with lower shrink temperatures are preferred to reduce or eliminate deformation of the first and the second tubular members during the welding process. In one example, suitable polymers for the compression sleeve 22 include FEP materials available from Optinova, Mariehamn, Finland, that have a shrink temperature of less than about 100 °C, or less than about 90 °C, or less than about 85 °C.

In some examples, compression sleeve 22 may have a thickness within a range from about 0.02 inches to about 0.05 inches (about 0.5 mm to about 1.27 mm), such as within a range from about 0.02 inches to about 0.03 inches (about 0.5 mm to about 0.76 mm).

As shown in FIG. 1A, in some examples first tubular member 18 and second tubular member 20 may be held together via compression sleeve 22. During a welding process, compression sleeve 22 is configured to compress against at least a portion of first tubular member 18, which causes pressure between compression sleeve 22 and first tubular member 18. First tubular member 18 further compresses against at least a portion of second tubular member 20, which causes pressure between first tubular member 18 and second tubular member 20. The pressure between compression sleeve 22 and first tubular member 18 and the pressure between first tubular member 18 and second tubular member 20 helps to hold first tubular member 18, second tubular member 20, and compression sleeve 22 in a substantially fixed position. In some examples, compression sleeve 22 may be configured to compress against at least a portion of second tubular member 20 to causes pressure between compression sleeve 22 and second tubular member 20.

In some examples, the materials of the first tubular member 18, the second tubular member 20, or both, may be sufficiently dimensionally stable that the compression sleeve 22 is not needed to maintain the relative positions of the tubular members during the bonding process. For example, if the second tubular member 20 fits tightly in the lumen of the first tubular member such that there is minimal (or no) relative movement between the first tubular member 18 and the second tubular member 20 during a bonding procedure, the compression sleeve 22 may not be required over the first tubular member 18 to form a bond. In another example, if the polymeric material from which the first tubular member 18 is formed is sufficiently rigid, the compression member 22 may not apply sufficient compressive force against the first tubular member to cause deformation of the first tubular member, and the compression member 22 may not be required during a bonding procedure.

System 10 includes energy source 12, which emits energy beam 16. Energy source 12 may include, for example, a laser source. In some examples, energy source 12 may include a fiber laser such as a thulium fiber laser. Energy beam 16 may provide energy to thermally weld first tubular member 18 to second tubular member 20 of the medical device at a joint region 24.

System 10 may include at least one optical component that directs and/or focuses energy beam 16 to joint region 24. For example, the system 10 may include lens 14, which is positioned to directed energy beam 16 to joint region 24. In some examples, system 10 may include other optical components (e.g., lenses, collimators, or the like) and/or a fiber-optic beam delivery (FOBD) system. A FOBD system uses an optical cable to deliver energy beam 16 to joint region 24, enabling energy source 12 to be located remotely from first tubular member 18 and second tubular member 20 of the medical device during welding, if desired. FOBD systems may be configured to permit the output of one laser source to supply the laser energy to be used for several welding processes located in different locations.

System 10 may be configured to operate in a direct welding mode or a transmission welding mode to thermally weld first tubular member 18 to second tubular member 20 of the medical device.

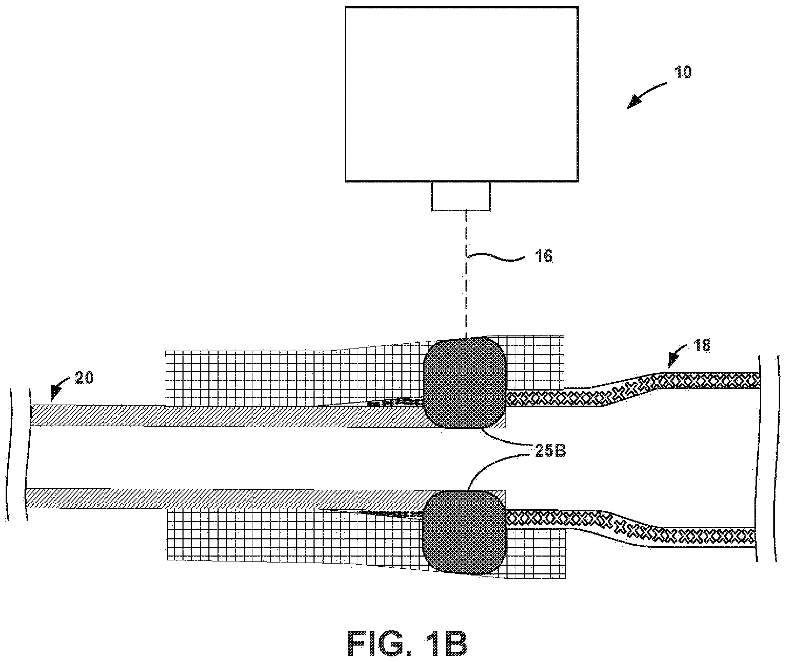

FIG. 1B is a conceptual diagram that illustrates an example of welding system 10 operating in a direct welding mode. When operating in the direct welding mode, an energy beam 16 may heat first tubular member 18 and second tubular member 20 directly to create a melt zone 25B that joins first tubular member 18 and second tubular member 20 together.

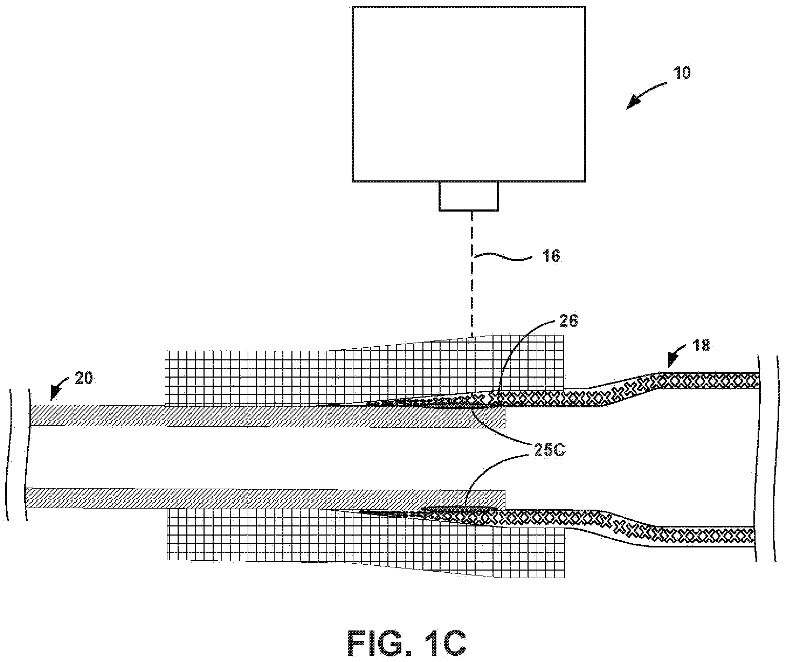

FIG. 1C is a conceptual diagram that illustrates an example of welding system 10 operating in a transmission welding mode. When operating in the transmission welding mode, energy beam 16 may be applied at interface 26 between first tubular member 18 and a second tubular member 20 with different transparencies to laser wavelengths to create a melt zone 25C that joins first tubular member 18 and second tubular member 20 together.

In some examples, system 10 may be configured to operate in a transmission welding mode to thermally weld first tubular member 18 to second tubular member 20 of the medical device. When operating in the transmission welding mode, energy source 12 may be configured to generate energy beam 16 with a selected wavelength of radiation transmittable through compression sleeve 22 and first tubular member 18, and absorbable by first tubular member 18 and second tubular member 20. In this way, energy beam 16 with the selected wavelength may be directed to joint region 24, e.g., interface 26 between first tubular member 18 and second tubular member 20. By directing energy beam 16 to interface 26, system 10 may be configured to weld both relatively thicker first tubular member 18 and/or relatively thicker second tubular member 20 compared to other thermal welding techniques, such as, for example, direct welding techniques or welding techniques using carbon dioxide lasers. Absorption of energy beam 16 at interface 26 heats portions of first tubular member 18 and/or second tubular member 20 to a softened state or molten state. Upon cooling, the softened or the molten portions of first tubular member 18 and/or second tubular member 20 solidify. The resulting joint region 24 provides mechanical coupling between first tubular member 18 and second tubular member 20.

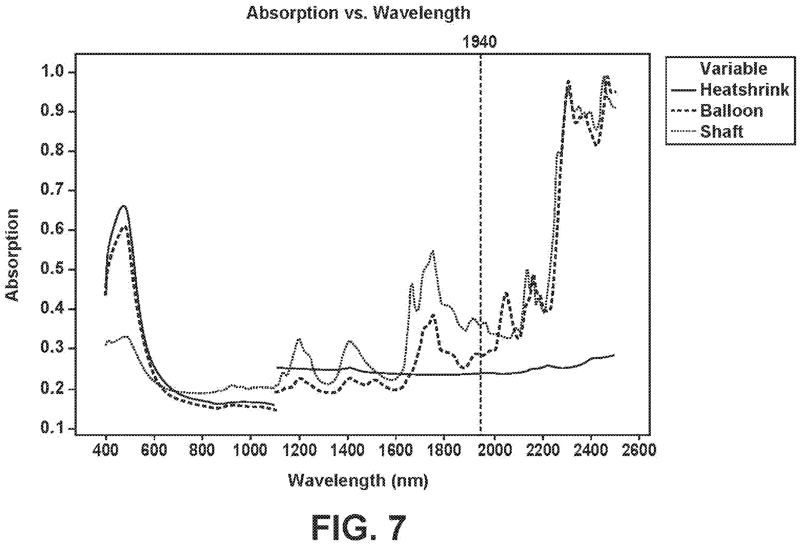

In accordance with aspects of this disclosure, energy source 12 may be configured to generate an energy beam 16 with a wavelength within a selected range to thermally weld both relatively thick parts and relatively thin parts of first tubular member 18 and second tubular member 20 together. In examples in which energy source 12 includes a fiber laser, the wavelength of energy beam 16 is within a range from about 1500 nm to about 2200 nm. In some examples, which are not intended to be limiting, the thulium fiber laser has an output wavelength of about 1940 nm to provide optimal heating with an FEP compression member 22. FIG. 7 is an example plot, which is not intended to be limiting and provided as an illustration, of absorption vs. wavelength for various components of a medical device.

In some examples, energy beam 16 may be directed through lens 14. Lens 14 may include any suitable type of lens, such as, for example, a collimating lens, a plano-convex lens, an aspheric lens, a cylinder lens, a laser generator lens, or the like. Lens 14 may be configured to direct energy beam 16 toward joint region 24 to weld first tubular member 18 and second tubular member 20 together. In some examples, lens 14 may be configured to focus energy beam 16 at or near interface 26, e.g., within tolerances of laser optics and/or thermal welding techniques. In some examples, as needed to form bonds between various tubular members 18, 20 with different thicknesses, shapes, beam absorption, and the like, the optical components may be used to shape the beam profile of the energy beam 16 to provide more concentrated heating in a small area, or less concentrated heating over a larger area.

Although direct welding may be used to weld first tubular member 18 to second tubular member 20, direct welding may result in a relatively thin weld joint at joint region 24, which may result in a relatively weak bond when welding relatively thick members. For instance, welding a relatively thick first tubular member 18 to a relatively thick second tubular member 20 may result in joint region 24 having a thickness less than first tubular member 18 and second tubular member 20. Additionally, direct welding requires a protective layer to be disposed over first tubular member 18 to protect first tubular member 18 and second tubular member 20.

The techniques of this disclosure may improve the welding process of medical devices. According to aspects of this disclosure, system 10 may be operated in transmission welding mode to weld first tubular member 18 and second tubular member 20 having both thick and thin parts, such as welding a catheter body with a medical balloon with high equator-to-neck ratio. Using transmission welding, system 10 may weld first tubular member 18 with second tubular member 20 while providing a smooth joint region 24 and a strong, hermetic joint between the two tubular members, e.g., to couple the first member and the second member via transmission welding. Additionally, transmission welding may eliminate the need to use a protective layer.

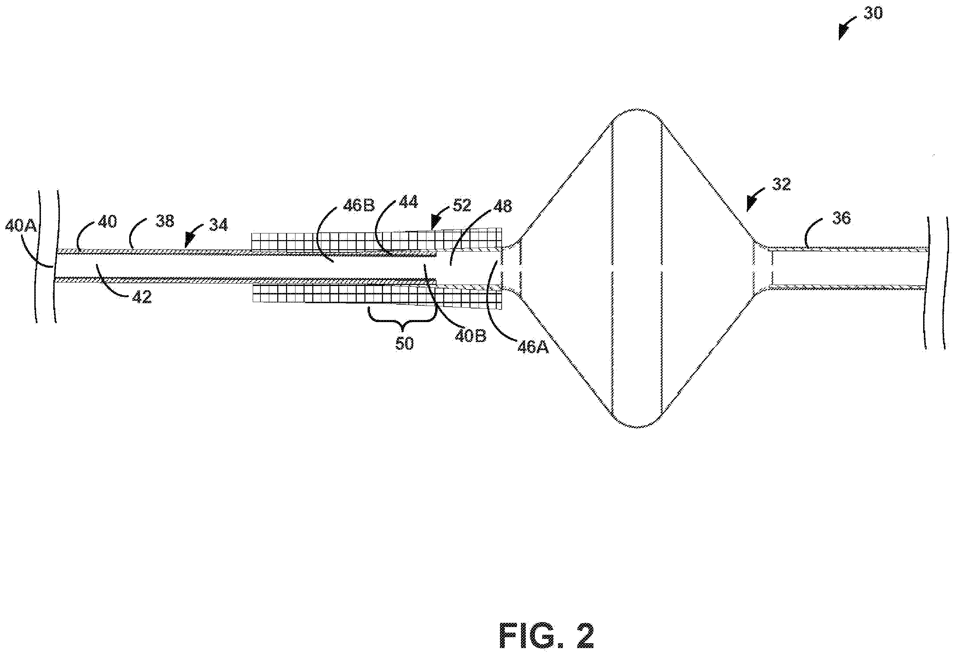

FIG. 2 is a conceptual and schematic diagram illustrating a view of an example medical device 30, including a first tubular member 32 and a second tubular member 34. First tubular member 32 may include a balloon 36. Second tubular member 34 may include a catheter 38. Balloon 36 may be thermally welded to catheter 38 adjacent to a distal end 40B of catheter 38.

Catheter 38 includes catheter body 40. Catheter body 40 extends from a proximal end 40A to the distal end 40B and defines a lumen 42. In some examples, catheter body 40 includes a tubular body. Catheter body 40 has a suitable length for accessing a target tissue site within the patient from a vascular access point. The length may be measured along a central longitudinal axis of catheter body 40. In some examples, catheter body 40 has a length within a range from about 80 cm to about 150 cm.

Catheter body 40 can be relatively thin-walled, such that it defines a relatively large inner diameter for a given outer diameter. For example, in some examples, an outer diameter of catheter body 40 may be about 3 French. The measurement term French, abbreviated Fr or F, is three times the diameter of a device as measured in mm. Thus, a 6 French diameter is about 2 millimeters (mm), a 5 French diameter is about 1.67 mm, a 4 French diameter is about 1.33 mm, and a 3 French diameter is about 1 mm. The term "about" as used herein with dimensions may refer to the exact value of the such as when used to describe numerical values, "about" or "approximately" refers to a range within the numerical value resulting from manufacturing tolerances and/or within 1%, 5%, or 10% of the numerical value. For example, a length of about 10 mm refers to a length of 10 mm to the extent permitted by manufacturing tolerances, or a length of 10 mm +/- 0.1 mm, +/- 0.5 mm, or +/- 1 mm in various examples.

Balloon 36 can be configured (e.g., sized and shaped) for any suitable medical procedure. In some examples, balloon 36 may include a high equator-to-neck ratio balloon. For example, balloon 36 can be configured to be inflated to facilitate a pulmonary vein isolation for treating atrial fibrillation. Balloon 36 can have any suitable length. In some examples, balloon 36 has a length of about 10 mm to about 300 mm.

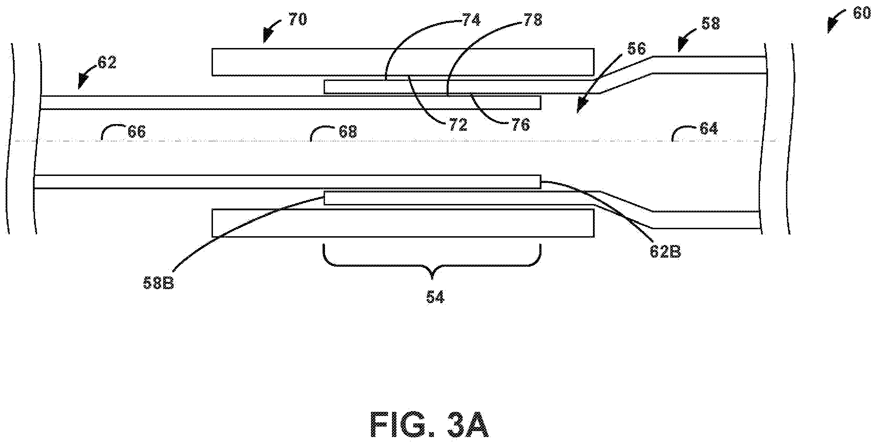

Balloon 36 can be relatively thin-walled, such that it defines a relatively large inner diameter for a given outer diameter. In some examples, the thickness of a wall 44 of balloon 36 may be substantially constant from a proximal end 46A to a distal end 46B. For example, lumen 48 may have a thickness of about 0.00762 mm to about 0.254 mm. In other examples, the thickness of wall 44 may taper from a first thickness at a proximal portion that includes proximal end 46A to a second thickness at a distal portion that includes distal end 46B, the second thickness being smaller than the first thickness. (as shown in FIG. 3A) For example, the thickness of wall 44 may taper from a first thickness of about 0.00762 mm to a second thickness of about to 0.254 mm.

A lumen 48 of balloon 36 may receive distal end 40B of catheter body 40. The overlapped region of balloon 36 and catheter 38 forms a joint region 50, which is configured to receive an energy beam to thermally weld balloon 36 to catheter 38.

A compression sleeve 52 may be disposed over catheter body 40 at joint region 50 to constrain balloon 36 and catheter 38 in a substantially fixed position before welding and may be removed after welding. In some examples, compression sleeve 52 may include a tubular heat-shrinkable sleeve, such as a heat-shrinkable tube. In some examples, compression sleeve 52 may include a non-tubular heat-shrinkable sleeve, such as a heat-shrinkable wrap.

After welding, balloon 36 is connected to catheter 38. Balloon 36 is configured to expand from a collapsed configuration to an expanded configuration via an inflation fluid delivered to balloon 36 via lumen 42 of catheter body 40. Balloon 36 may be inflated to any suitable pressure via an inflation fluid (e.g., saline) delivered to the balloon.

Balloon 36 is a balloon with high equator to neck ratio. As illustrated in FIG. 2, balloon 36 having a relatively large equator and a relatively small balloon neck. The techniques of this disclosure may help to reduce and prevent expansion and distortion of balloon neck (e.g., distal end 46B) of balloon 36 during a welding process. According to aspects of this disclosure, a low-profile weld, e.g., tapered, joint and a strong, hermetic bond is formed between first tubular member 32 and second tubular member 34.

FIGS. 3A-3D are conceptual diagrams that illustrate cross-sections of a joint region 54 before heat-shrinking, after heat-shrinking and before welding, after welding before sleeve removal, and after sleeve removal, respectively. As illustrated in FIG. 3A, lumen 56 of first tubular member 58 may receive at least a portion of second tubular member 62 (e.g., a distal end 62B of second tubular member 62) therein to define joint region 54. First tubular member 58 and second tubular member 62 are positioned such that a longitudinal axis 64 of first tubular member 58 and a longitudinal axis 66 of second tubular member 62 are aligned along a common axis 68. As longitudinal axes 64 and 66 are aligned and first and second tubular members 58 and 62 extend along common axis 68, a distal end 58B of first tubular member 58 overlaps distal end 62B of second tubular member 62 therein to define joint region 54.

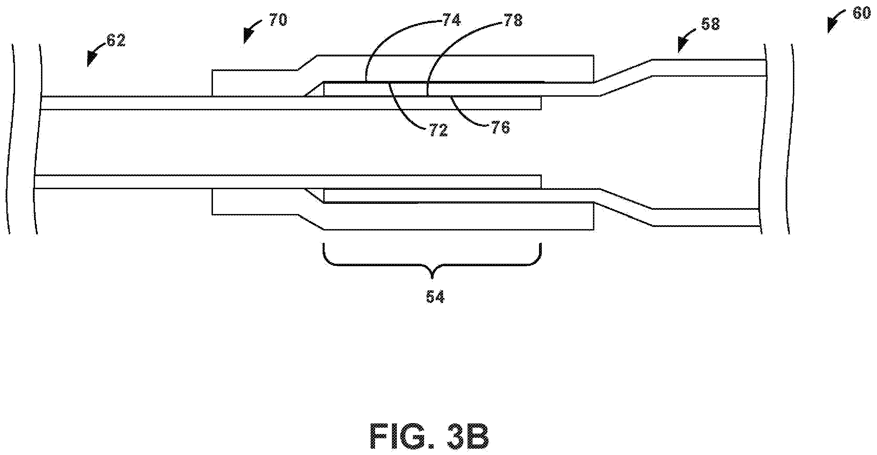

Compression sleeve 70 may define a lumen sized to receive at least a portion of first tubular member 58 (e.g., distal end 58B of first tubular member 58) at joint region 54. As illustrated in FIG. 3B, in example when compression sleeve 70 includes a heat-shrinkable sleeve, upon application of heat to compression sleeve 70, compression sleeve 70 may compress first tubular member 58 and/or second tubular member 62 at joint region 54. In examples in which sleeve 70 includes a cold-shrink tube, sleeve 70 may be configured to, after removal of a retainer coil, compress first tubular member 58 and/or second tubular member 62 at joint region 54. In examples in which sleeve 70 includes a compression wrap or clamp, sleeve 70 may be configured to, upon wrapping or clamping, compress first tubular member 58 and/or second tubular member 62 at joint region 54.

In some examples, compression of first tubular member 58 and second tubular member 62 at joint region 54 may hold first tubular member 58 and second tubular member 62 together during thermal welding. For example, compression of first tubular member 58 and second tubular member 62 at joint region 54 may cause a radially inner surface 72 of compression sleeve 70 compresses against a radially outer surface 74 of first tubular member 58, which causes pressure between inner surface 72 of compression sleeve 70 and outer surface 74 of first tubular member 58. Additionally, or alternative, a radially inner surface 76 of first tubular member 58 may compress against a radially outer surface 78 of second tubular member 62, to cause pressure between inner surface 76 of first tubular member 58 and outer surface 78 of second tubular member 62. The pressure between inner surface 72 of compression sleeve 70 and outer surface 74 of first tubular member 58 and/or the pressure between inner surface 76 of first tubular member 58 and outer surface 78 of second tubular member 62 may constrain first tubular member 58 and second tubular member 62 in a substantially fixed position.

During thermal welding, one or more energy beams may be directed and/or focused at joint region 54, e.g., outer surface 78 of second tubular member 62. Compression sleeve 70 is configured to compress first tubular member 58 and/or second tubular member 62 to form a tapered joint region 54. Additionally, or alternatively, compression sleeve 70 may constrain first tubular member 58 and second tubular member 62 maintain axial alignment and/or concentric alignment, e.g., relative to common axis 68, of first tubular member 58 and second tubular member 62.

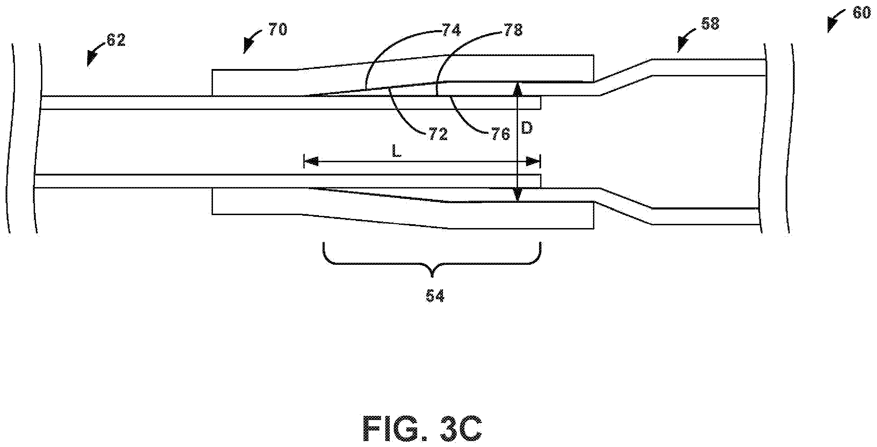

As illustrated in FIG. 3C, after thermal welding, a length L of the tapered joint region 54 may be within a range from about 0.05 inches (1.27 mm) to about 0.3 inches (7.62 mm), such as about 0.1 inches (2.54 mm) to about 0.2 inches (5.08 mm). Tapered joint region 54 may have a diameter D that tapers from a minimum to a maximum diameter. For example, the diameter D of tapered joint region 54 may be within a range from about 0.1 inches (2.54 mm) to about 0.2 inches (5.08 mm), such as about 0.12 inches (3.048 mm) to about 0.15 inches (3.81 mm).

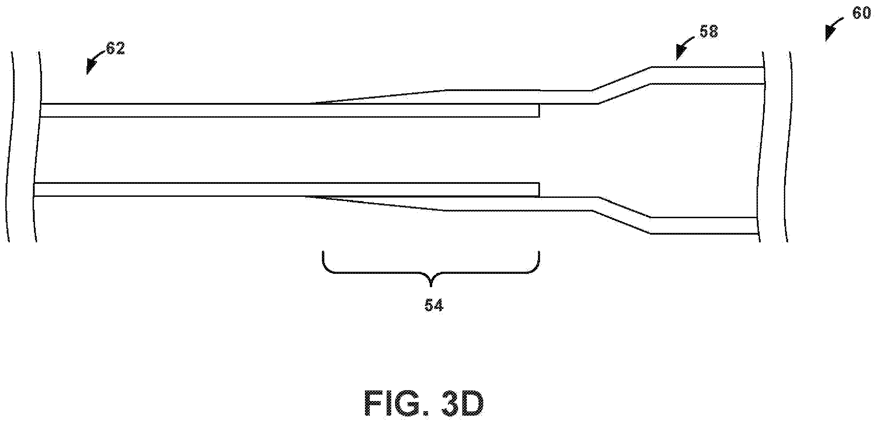

As illustrated in FIG. 3D, after thermal welding, compression sleeve 70 may be removed. Compression sleeve 70 may be removed by any suitable means, such as, for example, cutting, peeling, laser etching, or other material removal techniques. In some examples, joint region 54 may be ground and/or polished via any suitable means, to remove excess or undesired material from joint region 54.

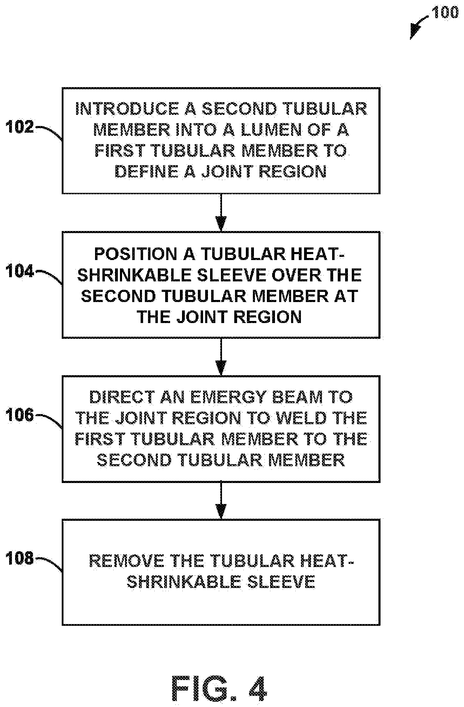

FIG. 4 is a flowchart illustrating an example method of welding a first tubular member to a second tubular member in accordance with aspects of this disclosure. The method 100 of FIG. 4 will be described with reference to FIG. 3A. While the steps of method 100 are described as occurring in a specific order in reference to FIG. 4, the order of steps is not limited to this example. For example, in other examples, a second energy beam may be applied after directing a first emery beam (106).

The technique illustrated in FIG. 4 includes introducing second tubular member 62 into lumen 56 of first tubular member 58 to defines joint region 54 (102). First tubular member 58 may comprise a first polymer and second tubular member 62 may comprise a second polymer. In some examples, first tubular member 58 includes a medical balloon with a high equator-to-neck ratio. In some examples, second tubular member 62 includes a catheter. In some examples, a first longitude axis 64 of first tubular member 58 and a second longitude axis 66 of second tubular member 62 are aligned along a common axis 68 to form joint region 54.

The technique illustrated in FIG. 4 includes positioning tubular compression sleeve 70 over at least a portion of first tubular member 58 at joint region 54 (104). In some examples, tubular compression sleeve 70 is formed of fluorinated ethylene propylene. Tubular compression sleeve 70 is configured to compress an inner surface 76 of first tubular member 58 against an outer surface 78 of second tubular member 62 when heated to a selected temperature. For example, the technique may include heating tubular compression sleeve 70 to shrink tubular compression sleeve 70, e.g., in a radial direction relative to common axis 68, to urge inner surface 76 of first tubular member 58 toward outer surface 78 of second tubular member 62. In examples in which sleeve 70 includes a cold-shrink tube, positioning sleeve 70 may include removing a retainer, such as a coil disposed on a radially interior surface of sleeve 70, to cause sleeve 70 to compress around first tubular member 58 and/or second tubular member 62 at joint region 54. In examples in which sleeve 70 includes a compression wrap, positioning sleeve 70 may include wrapping sleeve 70 around first tubular member 58 and/or second tubular member 62 at joint region 54. In examples in which sleeve 70 includes a clamp, positioning sleeve 70 may include moving the clamp from an expanded configuration toward a contracted configuration to compress first tubular member 58 and/or second tubular member 62 at joint region 54. In some examples, tubular compression sleeve 70 helps to constrain first tubular member 58 and second tubular member 62 in a substantially fixed position.

The technique illustrated in FIG. 4 includes, after positioning sleeve 70, e.g., once first tubular member 58 and second tubular member 62 are constrained in a substantially fixed position, directing an energy beam of a laser toward joint region 54 (106). In some examples, the laser may include a fiber laser, such as a thulium fiber laser. In response to absorption of at least a portion of the radiation of the energy beam, first tubular member 58 and/or second tubular member 62 may be heated to thermally weld first tubular member 58 and second tubular member 62 at joint region 54. In some examples, directing the energy beam may include selecting a duration of time for directing energy beam toward joint region 54. For example, the duration of time may be selected based on a material of first tubular member 58 and/or second tubular member 62, a compression by sleeve 70, a wavelength of the energy beam, or other parameter affecting the temperature or rate of temperature increase of first tubular member 58 and/or second tubular member 62. In some examples, directing the energy beam may include selecting a wavelength, or a wavelength range, of radiation of the energy beam. For example, selecting the wavelength may include selecting the wavelength of radiation to be transmittable through tubular compression sleeve 70 and/or first tubular member 58, and absorbable by first tubular member 58 and second tubular member 62. In some examples, selecting the wavelength may include selecting the wavelength of radiation to be within a range from about 1500 nm to about 2200 nm, or about 1940 nm.

In some examples, directing the energy beam may include directing a first energy beam toward joint region 54 and directing a second, different energy beam toward joint region 54. For example, the first energy beam may be directed toward joint region 54 at a first axial position of first tubular member 58 and/or second tubular member 62 having a first thickness, and the second energy beam may be directed toward joint region 54 at a second axial position of first tubular member 58 and/or second tubular member 62 having a second, different thickness. In some examples, directing the energy beam may include directing a plurality of energy beam toward joint region 54, each energy beam of the plurality of energy beams having a selected wavelength of radiation.

The technique illustrated in FIG. 4 includes, after thermally welding first tubular member 58 and second tubular member 62, removing compression sleeve 70 (108). In some examples, removing sleeve 70 may include cutting, peeling, tearing, or otherwise separating sleeve 70 from first tubular member 58 and second tubular member 62.

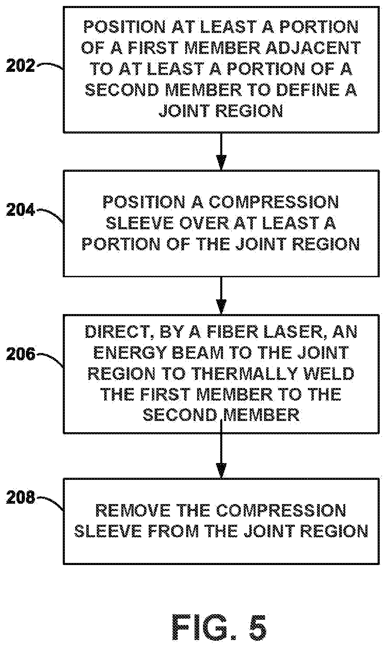

In some examples, the systems described herein may be used to thermally weld non-tubular polymeric components, such as polymeric sheets or other rectilinear or irregular shapes that are not tubular in shape. For example, FIG. 5 is a flowchart illustrating an example method of welding a first polymeric member to a second polymeric member in accordance with aspects of this disclosure.

The technique illustrated in FIG. 5 includes positioning at least a portion of a first member adjacent to at least a portion of a second member to define a joint region (202). The first member includes a first polymer. For example, the first member may be the same as or substantially similar to first tubular member 58 described above in reference to FIGS. 3A-3D, except that the first member may be non-tubular. The second member includes a second polymer. The second polymer is different than the first polymer. The second member may be the same as or substantially similar to second tubular member 62 described above in reference to FIGS. 3A-3D, except that the second member may be non-tubular.

The technique illustrated in FIG. 5 also includes positioning a compression sleeve over at least a portion of the joint region (204). The compression sleeve may be the same as or substantially similar to compression sleeve 70 described above in reference to FIGS. 3A-3D, except the compression sleeve may be configured to compress the non-tubular joint region.

The technique illustrated in FIG. 5 also includes directing, by a fiber laser, an energy beam to the joint region to thermally weld the first member to the second member (206). The fiber laser may be the same as or substantially similar to laser 12 described above in reference to FIG. 1A. For example, the energy beam includes a wavelength of radiation that is transmittable through the compression sleeve and the first member, and absorbable by the first member and the second member.

The technique illustrated in FIG. 5 optionally includes removing the compression sleeve from the joint region (208). For example, the sleeve may be removed by one or more of cutting, peeling, laser etching, or other material removal techniques.

### EXAMPLE

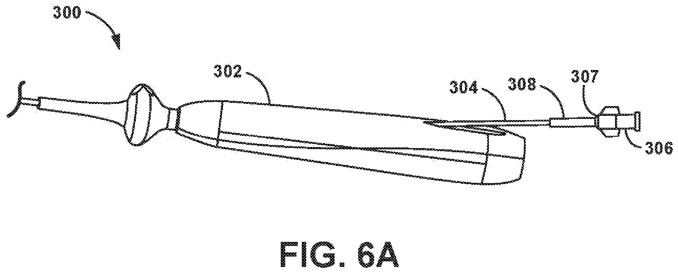

Referring now to FIG. 6A, a medical device 300 included a handle 302 and an irrigation tube 304. The irrigation tube 304 was to be bonded to a luer 306. The luer 306 included an orifice 307 with a diameter larger than an outside diameter of the irrigation tube 304. To form a fluid-tight fit between the irrigation tube 304 and the luer 306, an extension tube 308 was bonded to the irrigation tube 304 and the luer 306.

Attempts to bond the extension tube 308 to the irrigation tube 304, and the extension tube 308 to the luer 306, with adhesives resulted in formation that weakened the joints between the components and caused fluid leakage.

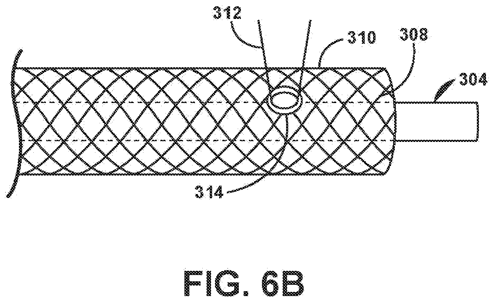

As shown in FIG. 6B, the irrigation tube 304 was inserted in a lumen in the extension tube 308, and a compression sleeve 310 was applied over the extension tube 308 to maintain the relative orientation of the extension tube 308 and the irrigation tube 304. A beam 312 from a thulium fiber laser at a wavelength of 1940 nm was focused at an interface 314 to shrink the compression sleeve 310 and weld the irrigation tube 304 and the extension tube 308.

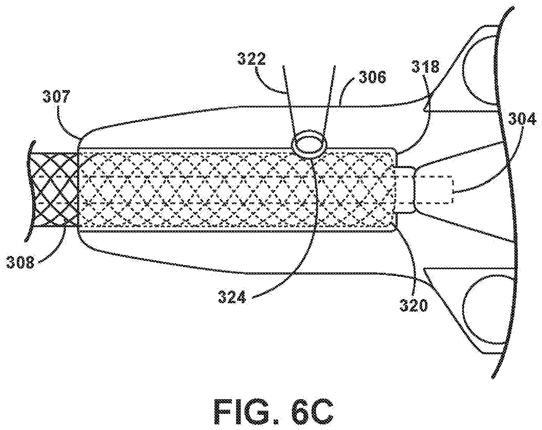

Referring now to FIG. 6C, following removal of the compression sleeve 310, the extension tube 308 was inserted into the orifice 307 in the luer 306 until a distal end 318 of the extension tube 308 abutted a wall 320 in the luer 306. The extension tube 308 was a tight friction fit in the opening 316, and the abutment of the extension tube 308 against the wall 320 prevented relative movement between the extension tube 308 and the luer 306.

A beam 322 from a thulium fiber laser at a wavelength of 1940 nm was focused at an interface 324 to bond the extension tube 308 and the luer 306.

Testing with an irrigation fluid revealed that the bonds between the irrigation tube 304 and the extension tube 308, and between the extension tube 308 and the luer 306, were liquid-tight under normal operating pressures.

Various examples have been described. These and other examples are within the scope of the following claims.

## Claims

1. A system for forming a medical device (50), comprising:
a first tubular member (18) comprising a first polymer;
a second tubular member (20) comprising a second polymer, wherein the first tubular member defines a lumen configured to receive at least a portion of the second tubular member therein to define a joint region (24);
a compression sleeve (22) configured to receive at least a portion of the first tubular member at the joint region; and
an energy source (12) comprising a fiber laser configured to deliver energy to the joint region to thermally weld the first tubular member to the second tubular member, wherein the energy comprises a wavelength of radiation transmittable through the sleeve and the first tubular member, and absorbable by the first tubular member and the second tubular member.

2. The system of claim 1, wherein the first tubular member comprises a medical balloon with a high equator-to-neck ratio, an extension tube (308) for a catheter body, or a luer (306).

3. The system of claims 1 or 2, wherein the second tubular member comprises a catheter body (38), a rigid hub, a luer (306), or an extension tube (308) on the catheter body.

4. The system of any of claims 1 to 3, wherein the compression sleeve comprises a heat-shrinkable sleeve comprising fluorinated ethylene propylene.

5. The system of any of claims 1 to 4, wherein the fiber laser comprises a thulium fiber laser.

6. The system of any of claims 1 to 5, wherein the wavelength of the fiber laser is within a range from about 1500 nm to about 2200 nm.

7. The system of any of claims 1 to 6, wherein the wavelength of the fiber laser is about 1940 nm.

8. The system of any of claims 1 to 7, wherein the compression sleeve is configured to compress an inner surface (76) of the first tubular member against an outer surface (78) of the second tubular member.

9. The system of any of claims 1 to 8, wherein the compression sleeve comprises a tubular heat-shrinkable sleeve configured to compress the joint region in response to thermal energy.

10. A method of forming a medical device (50), comprising:
preparing a joint region (24) by introducing at least a portion of a second tubular member (20) into a lumen of a first tubular member (18), wherein the first tubular member comprising a first polymer, wherein the second tubular member comprising a second polymer;
positioning a compression sleeve (22) over at least a portion of the first tubular member at the joint region;
directing, by a fiber laser, an energy beam (16) to the joint region to thermally weld the first tubular member to the second tubular member, wherein the energy beam comprises a wavelength of radiation transmittable through the compression sleeve and the first tubular member, and absorbable by the first tubular member and the second tubular member; and
removing the compression sleeve from the joint region.

11. The method of claim 10, wherein the first tubular member comprises a medical balloon with a high equator-to-neck ratio, an extension tube (308) for a catheter body, or a luer (306).

12. The method of claims 10 or 11, wherein the second tubular member comprises a catheter body (38), a rigid hub, a luer (306), or an extension tube (308)on the catheter body; and/or wherein the compression sleeve comprises a tubular heat-shrinkable sleeve comprising fluorinated ethylene propylene.

13. The method of any of claims 10 to 12, wherein the fiber laser comprises a thulium fiber laser; and optionally wherein directing the energy beam to the joint region further comprises selecting the wavelength of the fiber laser within a range from about 1500 nm to about 2200 nm.

14. The method of claim 13, wherein directing the energy beam to the joint region further comprises selecting the wavelength of the fiber laser of about 1940 nm.

15. The method of any of claims 10 to 14, wherein positioning the compression sleeve further comprises compressing, by the compression sleeve, an inner surface (76) of the first tubular member against an outer surface (78) of the second tubular member; and/or wherein preparing a joint region by introducing at least the portion of the second tubular member into the lumen of the first tubular member further comprises aligning a first longitude axis (64) of the first tubular member and a second longitude axis (66) of the second tubular member along a common axis (68) to form the joint region.

## Patentansprüche

1. System zum Ausbilden einer medizinischen Vorrichtung (50), umfassend:
ein erstes röhrenförmiges Element (18), umfassend ein erstes Polymer;
ein zweites röhrenförmiges Element (20), umfassend ein zweites Polymer, wobei das erste röhrenförmige Element ein Lumen definiert, das konfiguriert ist, um mindestens einen Abschnitt des zweiten röhrenförmigen Elements darin aufzunehmen, um einen Verbindungsbereich (24) zu definieren;
eine Kompressionshülse (22), die konfiguriert ist, um mindestens einen Abschnitt des ersten röhrenförmigen Elements in dem Verbindungsbereich aufzunehmen; und
eine Energiequelle (12), umfassend einen Faserlaser, der konfiguriert ist, um Energie an den Verbindungsbereich abzugeben, um das erste röhrenförmige Element mit dem zweiten röhrenförmigen Element thermisch zu verschweißen, wobei die Energie eine Strahlungswellenlänge umfasst, die durch die Hülse und das erste röhrenförmige Element transmittierbar ist und durch das erste röhrenförmige Element und das zweite röhrenförmige Element absorbierbar ist.

2. System nach Anspruch 1, wobei das erste röhrenförmige Element einen medizinischen Ballon mit einem hohen Äquator-zu-Hals-Verhältnis, einer Verlängerungsröhre (308) für einen Katheterkörper oder einen Luer (306) umfasst.

3. System nach Anspruch 1 oder 2, wobei das zweite röhrenförmige Element einen Katheterkörper (38), eine starre Nabe, einen Luer (306) oder eine Verlängerungsröhre (308) an dem Katheterkörper umfasst.

4. System nach einem der Ansprüche 1 bis 3, wobei die Kompressionshülse eine wärmeschrumpfbare Hülse umfasst, umfassend fluoriertes Ethylenpropylen.

5. System nach einem der Ansprüche 1 bis 4, wobei der Faserlaser ein Thuliumfaserlaser ist.

6. System nach einem der Ansprüche 1 bis 5, wobei die Wellenlänge des Faserlasers innerhalb eines Bereichs von etwa 1500 nm bis etwa 2200 nm liegt.

7. System nach einem der Ansprüche 1 bis 6, wobei die Wellenlänge des Faserlasers etwa 1940 nm beträgt.

8. System nach einem der Ansprüche 1 bis 7, wobei die Kompressionshülse konfiguriert ist, um eine Innenoberfläche (76) des ersten röhrenförmigen Elements gegen eine Außenoberfläche (78) des zweiten röhrenförmigen Elements zu komprimieren.

9. System nach einem der Ansprüche 1 bis 8, wobei die Kompressionshülse eine röhrenförmige wärmeschrumpfbare Hülse umfasst, die konfiguriert ist, um den Verbindungsbereich als Reaktion auf thermische Energie zu komprimieren.

10. Verfahren zum Ausbilden einer medizinischen Vorrichtung (50), umfassend:
Herstellen eines Verbindungsbereichs (24) durch Einführen mindestens eines Abschnitts eines zweiten röhrenförmigen Elements (20) in ein Lumen eines ersten röhrenförmigen Elements (18), wobei das erste röhrenförmige Element ein erstes Polymer umfasst, wobei das zweite röhrenförmige Element ein zweites Polymer umfasst;
Positionieren einer Kompressionshülse (22) über mindestens einem Abschnitt des ersten röhrenförmigen Elements in dem Verbindungsbereich;
Richten, durch einen Faserlaser, eines Energiestrahls (16) auf den Verbindungsbereich, um das erste röhrenförmige Element mit dem zweiten röhrenförmigen Element thermisch zu verschweißen, wobei der Energiestrahl eine Strahlungswellenlänge umfasst, die durch die Kompressionshülse und das erste röhrenförmige Element transmittierbar ist und durch das erste röhrenförmige Element und das zweite röhrenförmige Element absorbierbar ist; und
Entfernen der Kompressionshülse aus dem Verbindungsbereich.

11. Verfahren nach Anspruch 10, wobei das erste röhrenförmige Element einen medizinischen Ballon mit einem hohen Äquator-zu-Hals-Verhältnis, eine Verlängerungsröhre (308) für einen Katheterkörper oder einen Luer (306) umfasst.

12. Verfahren nach Anspruch 10 oder 11, wobei das zweite röhrenförmige Element einen Katheterkörper (38), eine starre Nabe, einen Luer (306) oder eine Verlängerungsröhre (308) an dem Katheterkörper umfasst; und/oder wobei die Kompressionshülse eine röhrenförmige wärmeschrumpfbare Hülse umfasst, umfassend fluoriertes Ethylenpropylen.

13. Verfahren nach einem der Ansprüche 10 bis 12, wobei der Faserlaser einen Thuliumfaserlaser umfasst; und gegebenenfalls wobei das Richten des Energiestrahls auf den Verbindungsbereich ferner ein Auswählen der Wellenlänge des Faserlasers innerhalb eines Bereichs von etwa 1500 nm bis etwa 2200 nm umfasst.

14. Verfahren nach Anspruch 13, wobei das Richten des Energiestrahls auf den Verbindungsbereich ferner das Auswählen der Wellenlänge des Faserlasers von etwa 1940 nm umfasst.

15. Verfahren nach einem der Ansprüche 10 bis 14, wobei das Positionieren der Kompressionshülse ferner das Komprimieren, durch die Kompressionshülse, einer Innenoberfläche (76) des ersten röhrenförmigen Elements gegen eine Außenoberfläche (78) des zweiten röhrenförmigen Elements umfasst; und/oder wobei das Herstellen eines Verbindungsbereichs durch Einführen mindestens des Abschnitts des zweiten röhrenförmigen Elements in das Lumen des ersten röhrenförmigen Elements ferner ein Ausrichten einer ersten Längsachse (64) des ersten röhrenförmigen Elements und einer zweiten Längsachse (66) des zweiten röhrenförmigen Elements entlang einer gemeinsamen Achse (68) umfasst, um den Verbindungsbereich auszubilden.

## Revendications

1. Système destiné à la formation d'un dispositif médical (50), comprenant :
un premier élément tubulaire (18) comprenant un premier polymère ;
un second élément tubulaire (20) comprenant un second polymère, dans lequel le premier élément tubulaire définit une lumière conçue pour recevoir au moins une partie du second élément tubulaire à l'intérieur de celle-ci pour définir une région de jonction (24) ;
un manchon de compression (22) conçu pour recevoir au moins une partie du premier élément tubulaire au niveau de la région de jonction ; et
une source d'énergie (12) comprenant un laser à fibre conçu pour délivrer de l'énergie à la région de jonction pour souder thermiquement le premier élément tubulaire au second élément tubulaire, dans lequel l'énergie comprend une longueur d'onde de rayonnement transmissible à travers le manchon et le premier élément tubulaire, et absorbable par le premier élément tubulaire et le second élément tubulaire.

2. Système selon la revendication 1, dans lequel le premier élément tubulaire comprend un ballon médical avec un rapport équateur/col élevé, un tube d'extension (308) pour un corps de cathéter, ou un Luer (306).

3. Système selon les revendications 1 ou 2, dans lequel le second élément tubulaire comprend un corps de cathéter (38), un moyeu rigide, un Luer (306), ou un tube d'extension (308) sur le corps de cathéter.

4. Système selon l'une quelconque des revendications 1 à 3, dans lequel le manchon de compression comprend un manchon thermorétractable comprenant de l'éthylène-propylène fluoré.

5. Système selon l'une quelconque des revendications 1 à 4, dans lequel le laser à fibre comprend un laser à fibre de thulium.

6. Système selon l'une quelconque des revendications 1 à 5, dans lequel la longueur d'onde du laser à fibre est au sein d'une plage d'environ 1 500 nm à environ 2 200 nm.

7. Système selon l'une quelconque des revendications 1 à 6, dans lequel la longueur d'onde du laser à fibre est d'environ 1 940 nm.

8. Système selon l'une quelconque des revendications 1 à 7, dans lequel le manchon de compression est conçu pour comprimer une surface interne (76) du premier élément tubulaire contre une surface externe (78) du second élément tubulaire.

9. Système selon l'une quelconque des revendications 1 à 8, dans lequel le manchon de compression comprend un manchon thermorétractable tubulaire conçu pour comprimer la région de jonction en réponse à de l'énergie thermique.

10. Procédé de formation d'un dispositif médical (50), comprenant :
la préparation d'une région de jonction (24) par l'introduction d'au moins une partie d'un second élément tubulaire (20) dans une lumière d'un premier élément tubulaire (18), dans lequel le premier élément tubulaire comprend un premier polymère, dans lequel le second élément tubulaire comprend un second polymère ;
le positionnement d'un manchon de compression (22) par-dessus au moins une partie du premier élément tubulaire au niveau de la région de jonction ;
l'orientation, par un laser à fibre, d'un faisceau d'énergie (16) vers la région de jonction pour souder thermiquement le premier élément tubulaire au second élément tubulaire, dans lequel le faisceau d'énergie comprend une longueur d'onde de rayonnement transmissible à travers le manchon de compression et le premier élément tubulaire, et absorbable par le premier élément tubulaire et le second élément tubulaire ; et
le retrait du manchon de compression de la région de jonction.

11. Procédé selon la revendication 10, dans lequel le premier élément tubulaire comprend un ballon médical avec un rapport équateur/col élevé, un tube d'extension (308) pour un corps de cathéter, ou un Luer (306).

12. Procédé selon les revendications 10 ou 11, dans lequel le second élément tubulaire comprend un corps de cathéter (38), un moyeu rigide, un Luer (306), ou un tube d'extension (308) sur le corps de cathéter ; et/ou dans lequel le manchon de compression comprend un manchon thermorétractable tubulaire comprenant de l'éthylène-propylène fluoré.

13. Procédé selon l'une quelconque des revendications 10 à 12, dans lequel le laser à fibre comprend un laser à fibre de thulium ; et éventuellement dans lequel l'orientation du faisceau d'énergie vers la région de jonction comprend en outre la sélection de la longueur d'onde du laser à fibre au sein d'une plage d'environ 1 500 nm à environ 2 200 nm.

14. Procédé selon la revendication 13, dans lequel l'orientation du faisceau d'énergie vers la région de jonction comprend en outre la sélection de la longueur d'onde du laser à fibre d'environ 1 940 nm.

15. Procédé selon l'une quelconque des revendications 10 à 14, dans lequel le positionnement du manchon de compression comprend en outre la compression, par le manchon de compression, d'une surface interne (76) du premier élément tubulaire contre une surface externe (78) du second élément tubulaire ; et/ou dans lequel la préparation d'une région de jonction par l'introduction au moins de la partie du second élément tubulaire dans la lumière du premier élément tubulaire comprend en outre l'alignement d'un premier axe de longitude (64) du premier élément tubulaire et d'un second axe de longitude (66) du second élément tubulaire le long d'un axe commun (68) pour former la région de jonction.
